(19) Europäisches Patentamt
European Patent Office
Office européen des brevets

(11) Veröffentlichungsnummer : **0 115 039**
**B1**

(12) # EUROPÄISCHE PATENTSCHRIFT

(45) Veröffentlichungstag der Patentschrift : **10.02.88**

(51) Int. Cl.⁴ : **C 07 D235/32**, A 61 K 31/415

(21) Anmeldenummer : **83112900.2**

(22) Anmeldetag : **21.12.83**

(54) **Substituierte Phenylsulfonyloxybenzimidazolcarbaminate, Verfahren zu ihrer Herstellung und ihre Verwendung als Arzneimittel.**

(30) Priorität : **23.12.82 DE 3247615**

(43) Veröffentlichungstag der Anmeldung : **08.08.84 Patentblatt 84/32**

(45) Bekanntmachung des Hinweises auf die Patenterteilung : **10.02.88 Patentblatt 88/06**

(84) Benannte Vertragsstaaten : **AT BE CH DE FR GB IT LI LU NL SE**

(56) Entgegenhaltungen : **DE-A- 2 441 201**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Rösner, Manfred, Dr.**
**Unter den Buchen 7**
**D-6239 Eppstein/Taunus (DE)**
Erfinder : **Loewe, Heinz, Dr.**
**Berliner Ring 6**
**D-6233 Kelkheim (Taunus) (DE)**
Erfinder : **Düwel, Dieter, Dr.**
**Frankfurter Strasse 38**
**D-6238 Hofheim am Taunus (DE)**
Erfinder : **Kirsch, Reinhard, Dr.**
**Kurfürstenstrasse 10**
**D-6239 Eppstein/Taunus (DE)**

**EP 0 115 039 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte Phenylsulfonyloxybenzimidazolcarbaminate, Verfahren zu ihrer Herstellung und die Verwendung als Arzneimittel, insbesondere als Anthelmintika.

Phenylsulfonyloxybenzimidazolcarbaminate mit anthelmintischen Wirkungen sind nach DE-OS 24 41 201 bekannt.

Es wurde nun eine neue Gruppe anthelmintisch wirksamer substituierter Phenylsulfonyloxybenzimidazolcarbaminate der Formel I gefunden

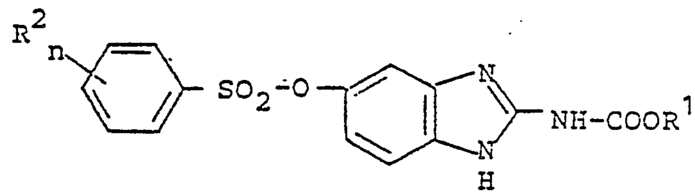

(I)

worin $R^1$ geradkettiges oder verzweigtes Alkyl mit 1-4 C-Atomen, n 1 oder 2 und die einzelnen Substituenten $R^2$ unabhängig voneinander Fluor, 1,1,2,2-Tetrafluorethoxy oder Cycloalkyl mit 3-8 C-Atomen bedeuten.

Unter den Verbindungen der Formel I sind solche bevorzugt, in denen $R^1$ Methyl, n 1 oder 2 und $R^2$ Fluor, 1,1,2,2-Tetrafluorethoxy oder Cycloalkyl mit 5, 6 oder 7 C-Atomen bedeuten.

Gegenstand der Erfindung sind weiterhin Verfahren zur Herstellung von substituierten Phenylsulfonyloxybenzimidazolcarbaminaten der Formel I, dadurch gekennzeichnet, daß man

a) ein o-Phenylendiaminderivat der Formel II

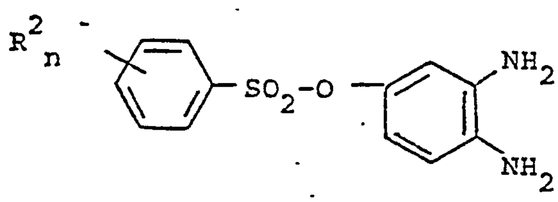

(II)

worin n und $R^2$ die zu Formel I angegebenen Bedeutungen haben, mit einem Carbaminsäureesterderivat der Formel III unter Ringschluß umsetzt

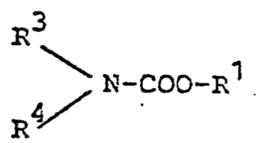

(III)

worin $R^1$ die zu Formel I angegebene Bedeutung hat und $R^3$ CN und $R^4$ Wasserstoff oder aber $R^3$ und $R^4$ zusammen einen der folgenden, doppelt an das Stickstoffatom gebundenen Reste

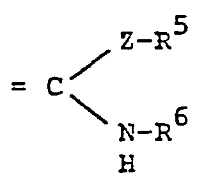 , 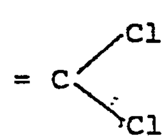 , 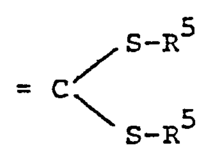

bedeuten, worin $R^5$ Alkyl mit 1-4 C-Atomen, $R^6$ Wasserstoff oder —$COOR^1$ und Z Sauerstoff oder Schwefel bedeuten, oder

b) ein Benzimidazolcarbaminat der Formel IV

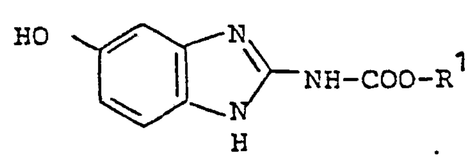

(IV)

mit einem substituierten Benzolsulfonsäurehalogenid der Formel V

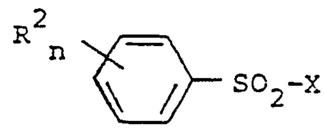

(V)

worin $R^1$, $R^2$ und n die zu Formel I angegebenen Bedeutungen haben und X Fluor, Chlor, Brom oder Jod bedeutet, umsetzt, oder

c) ein 2-Aminobenzimidazol der Formel VI

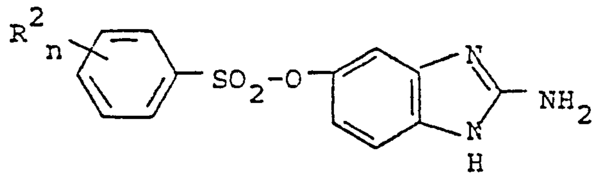

(VI)

mit einem Kohlensäureesterderivat der Formel VII

$$Y — COOR^1 \qquad (VII)$$

worin $R^1$, $R^2$ und n die oben angegebenen Bedeutungen haben und Y für $—OR^1$ oder Chlor steht, umsetzt.

Die als Ausgangsstoffe für das Verfahren a) verwendeten substituierten o-Phenylendiamine der Formel II werden durch Reduktion von Nitroaminoverbindungen der Formel VIII erhalten,

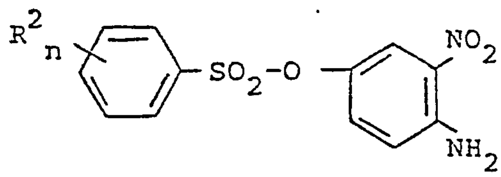

(VIII)

worin n und $R^2$ die oben angegebenen Bedeutungen haben.

Die Herstellung von Nitroaminen der Formel VIII ist in der DE-OS 24 41 201 beschrieben.

Sie erfolgt durch Umsetzung eines substituierten Benzolsulfonsäurehalogenids der Formel V mit 4-Amino-3-nitrophenol oder dessen Salzen in einem inerten Lösungsmittel wie z. B. Aceton in Gegenwart einer organischen oder anorganischen Base wie z. B. Triethylamin oder Natriumhydroxid. Die Verbindungen der Formel V sind entweder literaturbekannt, oder sie können in Analogie zu bekanten Verbindungen nach bekannten Verfahren hergestellt werden (vgl. Houben-Weyl, Methoden der Organischen Chemie, Band 9, S. 561-587, Stuttgart 1955).

Die Nitroaminoverbindungen der Formel VIII werden durch Reduktion, zweckmäßig mit katalytisch erregtem Wasserstoff, z. B. mit Raney-Nickel-, Platin- oder Palladium-Katalysatoren in einem Lösungsmittel wie Methanol, 2-Methoxyethanol oder Dimethylformamid oder deren Gemischen, bei Normaldruck oder unter einem Druck von 1-3 atü zu einer Diaminoverbindung der Formel II hydriert.

Zur Durchführung des Verfahrens a) wird ein Diamin der Formel II oder eines seiner Salze mit einer Verbindung der Formel III umgesetzt. Die Verbindungen der Formel III sind bekannt.

Wenn in Formel III $R^3$ CN und $R^4$ Wasserstoff bedeutet, versetzt man zweckmäßig eine wässrige Lösung des Cyanamidcarboxylats der Formel III mit einem o-Phenylendiaminderivat der Formel II, stellt durch Zugabe einer anorganischen oder organischen Säure, z. B. Salzsäure oder Eisessig, einen pH-Wert von 1-6, vorzugsweise von 2-5 ein und führt die Reaktion zweckmäßigerweise zwischen 20 und 100 °C durch. Je nach Reaktivität der Verbindung der Formel II liegt die Reaktionszeit zwischen 30 Minuten und 10 Stunden.

Wenn in Formel III $R^3$ und $R^4$ zusammen

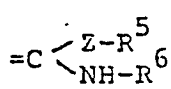

bedeuten, worin $R^5$, $R^6$ und Z die oben angegebenen Bedeutungen haben, erfolgt die Umsetzung mit o-Phenylendiaminen der Formel II in einem geeigneten Lösungsmittel in einem Temperaturbereich von 0 bis 150 °C, vorzugsweise bei der Siedetemperatur des Reaktionsgemisches, und unter Zusatz einer anorganischen oder organischen Säure wie Salzsäure, Schwefelsäure, Ameisensäure, Eisessig, p-Toluolsulfonsäure. Als Lösungsmittel kommen z. B. in Frage : Wasser, Methanol, Ethanol, Isopropanol, Amylalkohol, Glykolmonomethylether, Diaoxan, Tetrahydrofuran, Diethylether, Diisopropylether, Dimethoxyethan, Toluol, Xylol, Eisessig, Aceton, Butanon, Methylenchlorid, Chloroform. Die Reaktionszeiten

können je nach Art der Substituenten und Reaktionsbedingungen zwischen etwa 30 Minuten und 10 Stunden liegen.

Wenn $R^3$ und $R^4$ in Formel III zusammen

$$=C<\begin{matrix}Cl\\Cl\end{matrix}$$

bedeuten, setzt man den N-Dichlormethylencarbaminsäureester der Formel III und das o-Phenylendiaminderivat der Formel II zweckmäßigerweise in einem geeigneten Lösungsmittel in Gegenwart von mindestens zwei Äquivalenten einer anorganischen oder organischen Base wie z. B. Natriumhydroxid, Natriumhydrogencarbonat, Kaliumcarbonat, Triethylamin oder Pyridin um.

Als Lösungsmittel kommen z. B. Toluol, Xylol, Diethylether, Diisopropylether, Dioxan, Tetrahydrofuran, Dimethoxyethan, Methylenchlorid, Chloroform, Dichlorethan, Aceton oder Butanon in Frage. Man arbeitet bei — 20 bis + 80 °C, vorzugsweise zwischen 0 und 30 °C.

Wenn $R^3$ und $R^4$ in Formel III zusammen

$$=C<\begin{matrix}SR^5\\SR^5\end{matrix}$$

bedeuten, worin $R^5$ die oben gegebene Bedeutung hat, setzt man die Verbindung der Formel III mit einer Verbindung der Formel II, z. B. in Diisopropylether, Dioxan, Tetrahydrofuran, Dimethoxyethan, Chloroform, Dichlorethan, Toluol, Xylol, Aceton oder Butanon bei erhöhter Temperatur, zweckmäßig bei der Siedetemperatur des eingesetzten Lösungsmittels um.

Vorteilhaft reduziert man eine Nitroaminoverbindung der Formel VIII, wie beschrieben, zu einem substituierten o-Phenylendiamin II, trennt vom Katalysator ab, engt die Lösung des Diamins der Formel II unter vermindertem Druck ein und setzt den Rückstand direkt mit einer Verbindung der Formel III, wie beschrieben um zu einer Verbindung der Formel I.

Zur Durchführung des Verfahrens b) setzt man ein Hydroxybenzimidazolcarbaminat der Formel IV in einem aprotischen Lösungsmittel wie z. B. Aceton, Dioxan oder Dimethylformamid mit einem substituierten Benzolsulfonsäurehalogenid der Formel V in Anwesenheit einer organischen oder anorganischen Base wie Triethylamin, Kaliumcarbonat oder natriumhydroxid bei 0-50 °C um.

Die als Ausgangsstoffe für das Verfahren c) verwendeten 2-Aminobenzimidazole der Formel VI, worin die Substituenten die vorstehend genannten Bedeutungen haben, werden durch Umsetzen eines o-Phenylendiaminderivates der Formel II mit Bromcyan oder Cyanamid in Salzsäure in einem Lösungsmittel wie Wasser, Ethanol oder Dioxan erhalten.

Zur Durchführung des Verfahrens c) wird ein 2-Aminoabenzimidazolderivat der Formel VI zweckmäßig mit einem Dialkylcarbonat der Formel VII in einem Lösungsmitel wie z. B. Methanol, Ethanol oder in einem Überschuß des Dialkylcarbonats in Anwesenheit einer starken Base wie z. B. Natrium(m)ethylat oder Natriumhydrid umgesetzt. Die Reaktionstemperatur kann dabei zwischen — 20 und + 150 °C, vorzugsweise zwischen + 20 und + 100 °C liegen.

Die neuen substituierten Phenylsulfonyloxybenzimidazolcarbaminate gemäß der Erfindung sind wertvolle Chemotherapeutika und eignen sich zur Bekämpfung von parasitären Erkrankungen an Mensch und Tier.

Sie sind insbesondere wirksam gegen eine große Anzahl von Helminthen, z. B. Haemonchus, Trichostrongylus, Ostertagia, Cooperia, Chabertia, Strongyloides, Oesophagostomum, Hyostrongylus, Ancylostoma, Ascaris und Heterakis sowie Fasciola. Besonderes ausgeprägt ist die Wirksamkeit gegenüber Magen-Darmstrongyliden, Lungenwürmern und Leberegeln, von denen vor alem Haus- und Nutztiere befallen werden. Deshalb werden die Vervindungen gemäß der Erfindung insbesondere in Tierarzneimitteln verwendet.

Die Verbindungen der Formel I werden je nach Lage des Falles in Dosierungen zwischen 0,1 und 50 mg pro kg Körpergewicht 1 bis 14 Tage lang verabreicht.

Zur oralen Applikation kommen Tabletten, Dragees, Kapseln, Pulver, Granulate, Suspensionen oder Pasten in Betracht, welche die Wirkstoffe zusammen mit üblichen Hilfs- oder Trägerstoffen wie Stärke, Cellulosepulver, Talcum, Magnesiumstearat, Zucker, Gelatine, Calciumcarbonat, feinverteilter Kieselsäure, Carboxymethylcellulose oder ähnlichen Stoffen enthalten.

Die Verfahrensprodukte sind nicht nur oral appliziert ausgezeichnet wirksam, sondern können auch parenteral appliziert werden. Zur parenteralen Applikation kommen Lösungen in Betracht, z. B. ölige Lösungen, die unter Verwendung von Sesamöl, Olivenöl oder synthetischen Triglyceriden, ggf. mit einem Zusatz wie z. B. von Tokopherol als Antioxidans und/oder unter Verwendung von grenzflächenaktiven Stoffen wie Sorbitanfettsäureester hergestellt werden. Daneben kommen wäßrige Suspensionen in Betracht, die unter Verwendung von ethoxylierten Sorbitanfettsäureestern, ggf. unter Zusatz von Verdickungsmitteln, wie Polyethylenglykol oder Carboxymethylcellulose, hergestellt werden.

Die Konzentration der Wirkstoffe gemäß der Erfindung in den damit hergestellten Präparaten liegen

vorzugsweise für den Gebrauch als Veterinärarzneimittel zwischen 0,5 und 25 Gewichtsprozent ; für den Gebrauch als Humanarzneimittel liegen die Konzentrationen der Wirkstoffe vorzugsweise zwischen 20 und 80 Gewichtsprozent.

Die erfindungsgemäßen Verbindungen der Formel I zeigen überraschenderweise eine wesentlich stärkere und breitere anthelmintische Wirksamkeit als die aus der DE-OS 24 41 201 bekannten Verbindungen, insbesondere gegen Fasciola hepatica. So wirken z. B. die Verbindungen der nachstehenden Beispiele 1-3 schon mit weniger als 30 mg/kg p.o. gegen Fasciola hepatica am Schaf und am Rind mit über 90 % während die beste erfindungsgemäße Verbindung aus DE-OS 24 41 201, Beispiel 22, beim Rind in diesem Dosisbereich keine Wirkung zeigt.

Verfahren a)

Beispiel 1

5-(4-Fluorphenylsulfonyloxy) benzimidazol-2-carbaminsäuremethylester

15.6 g 2-Amino-5-(4-fluorphenylsulfonyloxy) nitrobenzol (Fp. 161 °C) werden in 75 ml Methanol und 75 ml Dimethylformamid mit einer katalytischen Menge Raney-Nickel bei Normaldruck hydriert. Nach beendeter Wasserstoffaufnahme wird der Katalysator abgesaugt, mit Methanol gewaschen und unter vermindertem Druck eingeengt.

Das entstandene 1,2-Diamino-4-(4-fluorphenylsulfonyloxy)-benzol wird in 150 ml Methanol und 25 ml Eisessig gelöst. Nach Zugabe von 15,5 g N,N'-Bis(methoxycarbonyl)-S-methyl-isothioharnstoff wird unter Rühren drei Stunden zum Rückfluß erhitzt. Die bereits nach ca. 10 Minuten einsetzende Fällung wird nach dem Abkühlen abgesaugt und mit Essigester gewaschen. Zur Reinigung löst man heiß in Dimethylformamid, versetzt mit Methanol und saugt das Produkt nach dem Abkühlen ab, Fp. 236 °C Zers.

In analoger Verfahrensweise werden mit entsprechend geänderten Ausgangsprodukten die folgenden erfindungsgemäßen Verbindungen der Formel I erhalten :

2. Aus 2-Amino-5-[3-(1,1,2,2-tetrafluorethoxy) phenylsulfonyloxy]-nitrobenzol (Fp. : Harz) über 1,2-Diamino-4-[3-(1,1,2,2-tetrafluorethoxy) phenylsulfonyloxy] benzol den 5-[3-(1,1,2,2-Tetrafluorethoxy) phenylsulfonyloxy]-benzimidazol-2-carbaminsäuremethylester, Fp. 186 °C.

3. Aus 2-Amino-5-(4-cyclohexylphenylsulfonyloxy) nitrobenzol (Fp. 123 °C) über 1,2-Diamino-4-(4-cyclohexylphenylsulfonyloxy) benzon den 5-(4-Cyclohexylphenylsulfonyloxy)-benzimidazol-2-carbaminsäuremethylester, Fp. 254 °C Zers.

4. Aus 2-Amino-5-(3-fluorphenylsulfonyloxy) nitrobenzol (Fp. 148 °C) über 1,2-Diamino-4-(3-fluorphenylsulfonyloxy) benzol den 5-(3-Fluorphenylsulfonyloxy) benzimidazol-2-carbaminsäuremethylester, Fp. 233 °C Zers.

5. Aus 2-Amino-5-(3,4-difluorphenylsulfonyloxy) nitrobenzol (Fp. 133 °C) über 1,2-Diamino-4-(3,4-difluorphenylsulfonyloxy) benzol den 5-(3,4-Difluorphenylsulfonyloxy)-benzimidazol-2-carbaminsäuremethylester, Fp. 223 °C Zers.

6. Aus 2-Amino-5-(2,4-difluorphenylsulfonyloxy) nitrobenzol über 1,2-Diamino-4-(2,4-difluorphenylsulfonyloxy)-benzol den 5-(2,4-Difluorphenylsulfonyloxy) benzimidazol-2-carbaminsäuremethylester, Fp. 248 °C Zers.

7. Aus 2-Amino-5-(2-fluorphenylsulfonyloxy) nitrobenzol (Fp. 124 °C) über 1,2-Diamino-4-(2-fluorphenylsulfonyloxy)-benzol den 5-(2-Fluorphenylsulfonyloxy) benzimidazol-2-carbaminsäuremethylester, Fp. 254 °C Zers.

8. Aus 2-Amino-5-(4-cyclopentylphenysulfonyloxy) nitrobenzol über 1,2-Diamino-4-(4-cyclopentylphenylsulfonyloxy)-benzol den 5-(4-Cyclopentylphenylsulfonyloxy) benzimidazol-2-carbaminsäuremethylester, FP 187 °C Zers.

9. Aus 2-Amino-5-(4-cycloheptylphenylsulfonyloxy) nitrobenzol über 1,2-Diamino-4-(4-cycloheptylphenylsulfonyloxy)-benzol den 5-(4-Cycloheptylphenylsulfonyloxy) benzimidazol-2-carbaminsäuremethylester, Fp. 157 °C.

Beispiel 10

5-(4-Fluorphenylsulfonyloxy) benzimidazol-2-carbaminsäuremethylester

Zu einer Lösung von 4,2 g Cyanamid in 20 ml Wasser gibt man 9 g Chlorameisensäuremethylester une 20 ml 33 %ige Natronlauge. Danach rührt man die Mischung 1,5 Stunden bei 30-35 °C. Zu dieser Lösung gibt man eine Lösung von 22.6 g 1,2-Diamino-4-(4-fluorphenylsulfonyloxy) benzol (s. Beispiel 1) in 200 ml Isopropanol und steigert die Temperatur bis 80 °C. Nach Zugabe von 30 ml Eisessig erwärmt man das Reaktionsgemisch noch 3 bis 4 Stunden auf etwa 90 °C. Nach Erkalten wird das Produkt abgesaugt und mit Isopropanol und Wasser gewaschen. Zur Reinigung löst man heiß in Dimethylformamid, versetzt mit Methanol und saugt das Produkt nach dem Abkühlen, ab, Fp. 236 °C Zers.

Analog zu Beispiel 10 werden unter Verwendung entsprechend geänderter Ausgangsmaterialien,

auch die erfindungsgemäßen Verbindungen der Formel I aus den Beispielen 2 bis 9 erhalten.

Beispiel 11

5-[3-(1,1,2,2-Tetrafluorethoxy) phenylsulfonyloxy] benzimidazol-2-carbaminsäuremethylester

Eine Mischung aus 38 g 1,2-Diamino-4-[3-(1,1,2,2-tetrafluorethoxy)-phenylsulfonyloxy) benzol (s. Beispiel 2), 20,2 g Triethylamin und 300 ml Methylenchlorid wird unter Rühren langsam mit einer Lösung von 15,6 g N-Dichlormethylen-carbaminsäuremethylester in 50 ml Methylenchlorid bei einer Temperatur von ca. 20 °C versetzt. Man rührt eine Stunde nach, saugt den Niederschlag ab und wäscht ihn mit Diisopropylether. Zur Reinigung kristallisiert man aus Methanol um, Fp. 186 °C.

Analog zu Beispiel 11 werden unter Verwendung entsprechend geänderter Ausgangsmaterialien auch die erfindungsgemäßen Verbindungen der Formel I aus den Beispielen 1 und 3 bis 9 erhalten.

Verfahren b)

Beispiel 12

5-(4-Fluorphenylsulfonyloxy) benzimidazol-2-carbaminsäuremethylester

Zu 2,07 g 5-Hydroxybenzimidazol-2-carbaminsäuremethylester und 1,94 g 4-Fluorbenzolsulfochlorid tropft man unter Rühren bei etwa 10 °C 2,8 ml Triethylamin. Man rührt anschließend 2 h bei Raumtemperatur nach, gibt unter Kühlung 100 ml 2n-Essigsäure zu, saugt den entstandenen Niederschlag ab, wäscht mit Wasser und trocknet. Fp. 236 °C Zers., aus Dimethylformamid/Methanol.

Das Ausgangsmaterial wird wie folgt erhalten :

5-Hydroxybenzimidazol-2-carbaminsäuremethylester

7,7 g 4-Amino-3-nitrophenol werden in Methanol mit Raney-Nickel/$H_2$ bei Normaldruck hydriert. Nach beendeter Wasserstoffaufnahme wird der Katalysator abgesaugt und die Lösung auf etwa 150 ml eingeengt. Man gibt 25 ml Eisessig und 12,4 g N,N'-Bis(methoxycarbonyl)-S-methylisothioharnstoff zu und erhitzt unter Rühren 3 h zum Rückfluß. Die Lösung wird eingeengt, mit Diisopropylether verrührt und abgesaugt. Zur Reinigung kristallisiert man aus Essigester µm, Fp. 300 °C, beginnende Zers.

Analog zu Beispiel 12 werden unter Verwendung entsprechender substituierter Benzolsulfonsäurehalogenide der Formel V auch die erfindungsgemäßen Verbindungen der Formel I aus den Beispielen 2-9 erhalten.

Verfahren c)

Beispiel 13

5-(4-Fluorphenylsulfonyloxy) benzimidazol-2-carbaminsäuremethylester

1,1 g Natriummethylat in 25 ml absolutem Toluol werden mit 3,1 g 2-Amino-5-(4-fluorphenylsulfonyloxy) benzimidazol und 1.0 g Dimethylcarbonat unter Rühren langsam auf 120 °C erhitzt und 4 h bei dieser Temperatur belassen. Nach dem Abkühlen wird unter vermindertem Druck eingeengt, mit wasser versetzt und mit Methylenchlorid/Methanol (9 : 1 vol/vol) extrahiert. Nach dem Einengen wird mit dem gleichen Lösungsmittelgemisch an Kieselgel chromatografiert. Aus den entsprechenden Fraktionen erhält man das Produkt durch Ausfällung mit Diisopropylether. Man saugt ab, wäscht mit Diisopropylether und trocknet ; Fp. 236 °C Zers.

Das Ausgangssmaterial wird wie folgt erhalten :

2-Amino-5-(4-fluorphenylsulfonyloxy) benzimidazol

14,1 g 1,2-Diamino-4-(4-fluorphenylsulfonyloxy) benzol (s. Beispiel 1) und 5,8 g Bromcyan werden in 150 ml Methanol 6 h zum Rückfluß erhitzt, anschließend abgekühlt und eingeengt. Der Rückstand wird im Wasser gelöst und unter Kühlung mit Ammoniak versetzt. Das ausfallende 2-Aminobenzimidazolderivat wird abgesaugt, mit Wasser gewaschen, getrocknet und aus Essigsäureäthylester, Diisopropylether unter Zusatz von Aktivkohle umkristallisiert ; Fp. 192 °C.

Analog zu Beispiel 13 werden unter Verwendung entsprechender substituierter 2-Aminobenzimidazole der Formel VI auch die erfindungsgemäßen Verbindungen der Formel I aus den Beispielen 2-9 erhalten.

**Patentansprüche** (für die Vertragsstaaten : BE, CH, DE, FR, GB, IT, LI, LU, NL, SE)

1. Phenylsulfonyloxybenzimidazolcarbaminate der Formel I

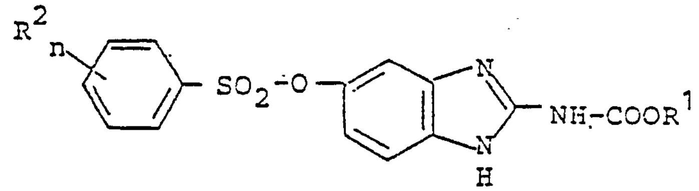

(I)

worin $R^1$ geradkettiges oder verzweigtes Alkyl mit 1-4 C-Atomen, n 1 oder 2 und die einzelnen Substituenten $R^2$ unabhängig voneinander Fluor, 1,1,2,2-Tetrafluorethoxy oder Cycloalkyl mit 3-8 C-Atomen bedeuten.

2. Verbindungen der Formel I von Anspruch 1, worin $R^1$ Methyl, n 1 oder 2, $R^2$ Fluor, 1,1,2,2-Tetrafluorethoxy oder Cycloalkyl mit 5, 6 oder 7 C-Atomen bedeuten.

3. Verfahren zur Herstellung von substituierten Phenylsulfonyloxybenzimidazolcarbaminaten der Formel I in Anspruch 1, dadurch gekennzeichnet, daß man

a) ein substituiertes o-Phenylendiaminderivat der Formel II

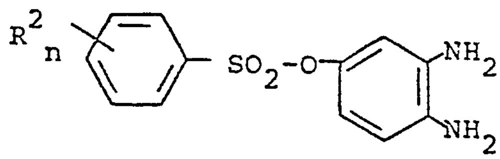

(II)

worin n und $R^2$ die zu Formel I in Anspruch 1 angegebenen Bedeutungen haben, mit einem Carbaminsäure-ester der Formel III

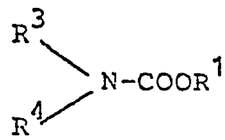

(III)

worin $R^1$ die zu Formel I in Anspruch 1 angegebenen Bedeutungen hat und $R^3$ CN und $R^4$ Wasserstoff, oder aber $R^3$ und $R^4$ zusammen einen der folgenden, doppelt an das Stickstoffatom gebundenen Reste bedeuten,

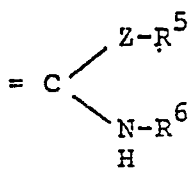 , 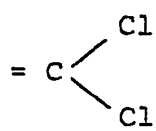 , 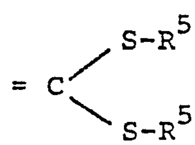

worin $R^5$ Alkyl mit 1-4 C-Atomen, $R^6$ Wasserstoff oder —$COOR^1$ and Z Sauerstoff oder Schwefel bedeuten, umsetzt, oder

b) ein Benzimidazolcarbaminat der Formel IV

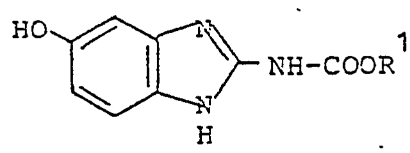

(IV)

mit einem substituierten Benzolsulfonsäurehalogenid der Formel V,

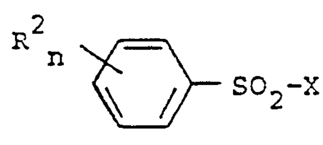

(V)

worin $R^1$, $R^2$ und n die zu Formel I in Anspruch 1 angegebenen Bedeutungen haben und X Fluor, Chlor,

Brom oder Jod bedeutet, umsetzt, oder

c) ein 2-Aminobenzimidazol der Formel VI,

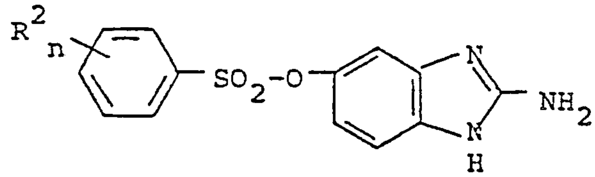

(VI)

mit einem Kohlensäureesterderivat der Formel VII,

$$Y{-}COOR^1 \qquad (VII)$$

worin $R^1$, $R^2$ und n die zu Formel I in Anspruch 1 angegebenen Bedeutungen haben und Y für $-OR^1$ oder Chlor steht, umsetzt.

4. Mittel zur Bekämpfung von Wurmerkrankungen, gekennzeichnet durch einen Gehalt an einem substituierten Phenylsulfonyloxybenzimidazolcarbaminat der Formel I in Anspruch 1.

5. Verbindung der Formel I von Anspruch 1, worin $R^1$ = Methyl und $(R^2)_n$ = 4-Fluor bedeuten.

6. Verbindung der Formel I von Anspruch 1, worin $R^1$ = Methyl und $(R^2)_n$ = 3-(1,1,2,2-Tetrafluorethoxy) bedeuten.

**Patentansprüche** (für den Vertragsstaat AT)

1. Verfahren zur Herstellung von substituierten Phenylsulfonyloxybenzimidazolcarbaminaten der Formel I

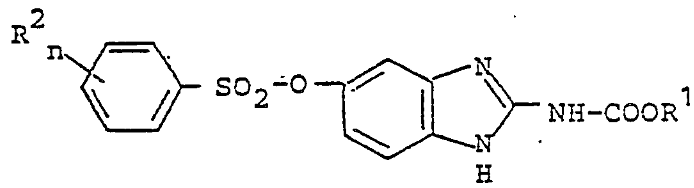

(I)

worin $R^1$ geradkettiges oder verzweigtes Alkyl mit 1-4 C-Atomen, n 1 oder 2 und die einzelnen Substituenten $R^2$ unabhängig voneinander Fluor, 1,1,2,2-Tetrafluorethoxy oder Cycloalkyl mit 3-8 C-Atomen bedeuten, dadurch gekennzeichnet, daß man

a) ein substituiertes o-Phenylendiaminderivat der Formel II

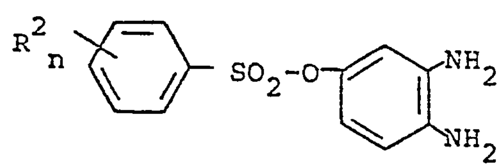

(II)

worin n und $R^2$ die zu Formel I in Anspruch 1 angegebenen Bedeutungen haben, mit einem Carbaminsäure-ester der Formel III

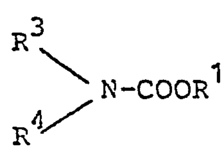

(III)

worin $R^1$ die zu Formel I in Anspruch 1 angegebenen Bedeutungen hat und $R^3$ CN und $R^4$ Wasserstoff, oder aber $R^3$ und $R^4$ zusammen einen der folgenden, doppelt an das Stickstoffatom gebundenen Reste bedeuten,

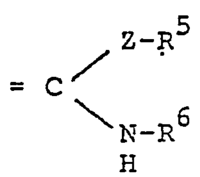 , 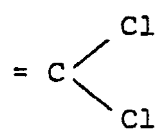 , 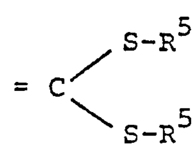

worin $R^5$ Alkyl mit 1-4 C-Atomen, $R^6$ Wasserstoff oder —$COOR^1$ und Z Sauerstoff oder Schwefel bedeuten, umsetzt, oder

b) ein Benzimidazolcarbaminat der Formel IV

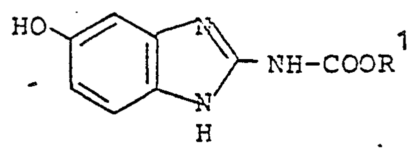

(IV)

mit einem substituierten Benzolsulfonsäurehalogenid der Formel V,

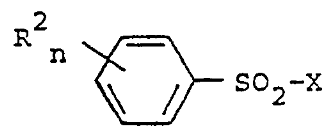

(V)

worin $R^1$, $R^2$ und n die zu Formel I in Anspruch 1 angegebenen Bedeutungen haben und X Fluor, Chlor, Brom oder Jod bedeutet, umsetzt, oder

c) ein 2-Aminobenzimidazol der Formel VI,

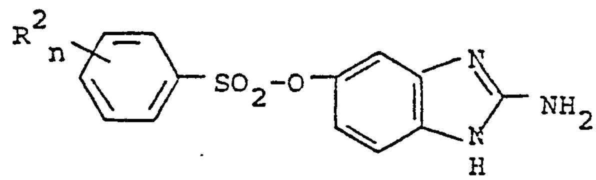

(VI)

mit einem Kohlensäureesterderivat der Formel VII,

$$Y—COOR^1 \quad (VII)$$

worin $R^1$, $R^2$ und n die zu Formel I in Anspruch 1 angegebenen Bedeutungen haben und Y für —$OR^1$ oder Chlor steht, umsetzt.

2. Verfahren gemäß Anspruch 1, dadurch gekennzeichnet, daß in den Formeln I bis III $R^1$ Methyl, $R^2$ Fluor, 1,1,2,2-Tetrafluorethoxy oder Cycloalkyl mit 5, 6 oder 7 C-Atomen bedeuten.

3. Verfahren gemäß Ansprüchen 1 oder 2, dadurch gekennzeichnet, daß in den Formeln I bis III $R^1$ Methyl und $(R^2)_n$ 4-Fluor bedeuten.

4. Verfahren gemäß Ansprüchen 1 oder 2, dadurch gekennzeichnet, daß in den Formeln I bis III $R^1$ Methyl und $(R^2)_n$ 3-(1,1,2,2-Tetrafluorethoxy) bedeuten.

**Claims** (for the Contracting States : BE, CH, LI, DE, FR, GB, IT, LU, NL, SE)

1. A phenylsulfonyloxybenzimidazolecarbamate of the formula I

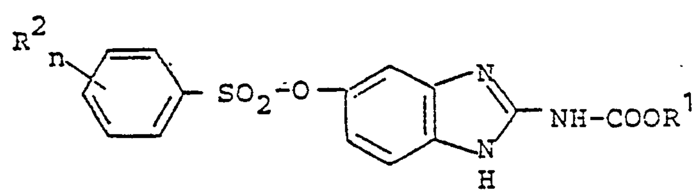

(I)

in which $R^1$ denotes straight-chain or branched alkyl having 1-4 carbon atoms, n denotes 1 or 2, and the individual substituents $R^2$, independently of one another, denote fluorine, 1,1,2,2-tetrafluoroethoxy or cycloalkyl having 3-8 carbon atoms.

2. A compound of the formula I in claim 1 in which $R^1$ denotes methyl, n denotes 1 and 2, and $R^2$ denotes fluorine, 1,1,2,2-tetrafluoroethoxy or cycloalkyl having 5, 6 or 7 carbon atoms.

3. A process for the preparation of substituted phenylsulfonyloxybenzimidazolecarbamates of the formula I in claim 1, which comprises

a) reacting a substituted o-phenylenediamine derivative of the formula II

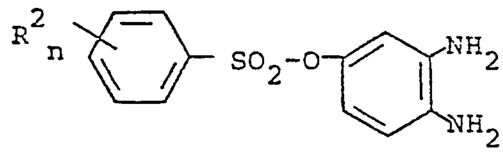

(II)

in which n and $R^2$ have the meanings indicated for formula I in claim 1, with a carbamic acid ester derivative of the formula III

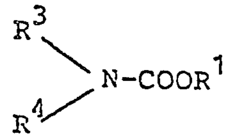

(III)

in which $R^1$ has the meaning indicated for formula I in claim 1, and $R^3$ denotes CN, and $R^4$ denotes hydrogen, or $R^3$ and $R^4$ together denote one of the following radicals double-bonded to the nitrogen atom

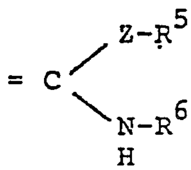 , 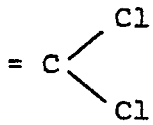 , 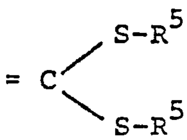

in which $R^5$ denotes alkyl having 1-4 carbon atoms, $R^6$ denotes hydrogen or $—COOR^1$, and Z denotes oxygen or sulfur, or

b) reacting a benzimidazolecarbamate of the formula IV

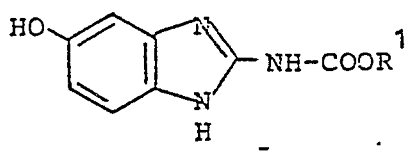

(IV)

with a substituted benzenesulfonyl halide of the formula V

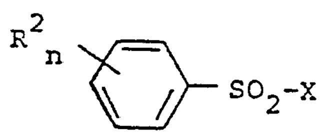

(V)

in which $R^1$, $R^2$ and n have the meanings indicated for formula I in claim 1, and X denotes fluorine, chlorine, bromine or iodine, or

c) reacting a 2-aminobenzimidazole of the formula VI

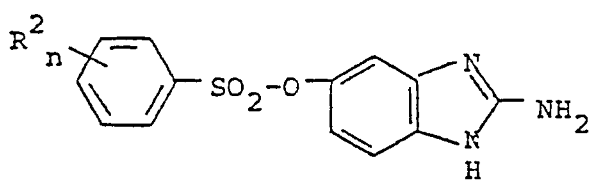

(VI)

with a carbonic ester derivative of the formula VII

$$Y—COOR^1 \qquad (VII)$$

in which $R^1$, $R^2$ and n have the meanings indicated in formula I in claim 1, and Y represents $—OR^1$ or chlorine.

4. Pharmaceutical composition for combatting helminths which contains a substituted phenylsulfonyloxybenzimidazole-carbamate of the formula I in claim 1.

5. A compound of the formula I in claim 1 in which $R^1$ denotes methyl and $(R^1)_n$ denotes 4-fluoro.

6. A compound of the formula I in claim 1 in which $R^1$ denotes methyl and $(R^2)_n$ denotes 3-(1,1,2,2-tetrafluoroethoxy).

**Claims** (for the Contracting State AT)

1. A process for the preparation of substituted phenylsulfonyloxybenzimidazolecarbamates of the formula I

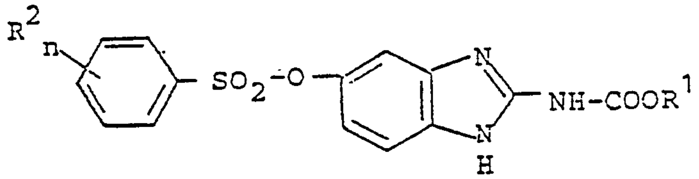

(I)

in which $R^1$ denotes straight-chain or branched alkyl having 1-4 carbon atoms, n denotes 1 or 2, and the individual substituents $R^2$, independently of one another, denote fluorine, 1,1,2,2-tetrafluoroethoxy of cycloalkyl having 3-8 carbon atoms, which comprises

a) reacting a substituted o-phenylenediamine derivative of the formula II

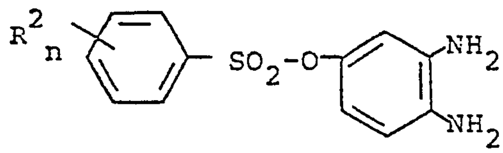

(II)

in which n and $R^2$ have the meanings indicated for formula I in claim 1, with a carbamic acid ester derivative of the formula III

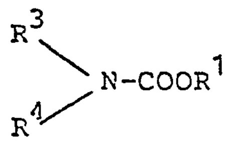

(III)

in which $R^1$ has the meaning indicated for formula I in claim 1, and $R^3$ denotes CN, and $R^4$ denotes hydrogen, or $R^3$ and $R^4$ together denote one of the following radicals double-bonded to the nitrogen atom

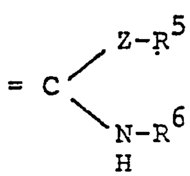 , 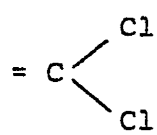 , 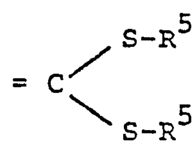

in which $R^5$ denotes alkyl having 1-4 carbon atoms, $R^6$ denotes hydrogen or —$COOR^1$, and Z denotes oxygen or sulfur, or

b) reacting a benzimidazolecarbamate of the formula IV

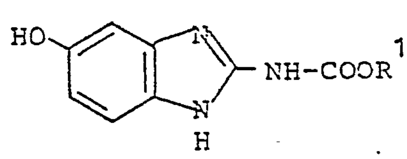

(IV)

with a substituted benzenesulfonyl halide of the formula V

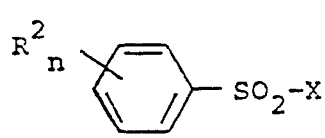

(V)

in which $R^1$, $R^2$ and n have the meanings indicated for formula I in claim 1, and X denotes fluorine, chlorine, bromine or iodine, or

c) reacting a 2-aminobenzimidazole of the formula VI

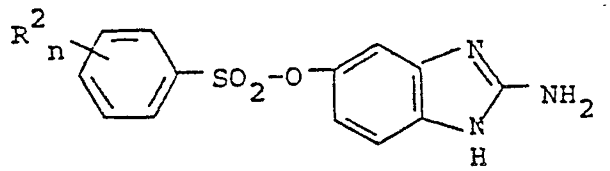

(VI)

with a carbonic ester derivative of the formula VII

$$Y—COOR^1 \qquad (VII)$$

in which $R^1$, $R^2$ and n have the meanings indicated in formula I in claim 1, and Y represents $—OR^1$ or chlorine.

2. A process according to claim 1 comprising formula I to III in which $R^1$ denotes methyl, $R^2$ denotes fluorine, 1,1,2,2-tetrafluoroethoxy or cycloalkyl havig 5, 6 oder 7 carbon atoms.

3. A process according to claim 1 or 2 comprising formula I to III in which $R^1$ denotes methyl and $(R^2)_n$ denotes 4-fluoro.

4. A process according to claim 1 or 2 comprising formula I to III in which $R^1$ denotes methyl and $(R^2)_n$ denotes 3-(1,1,2,2-tetrafluoroethoxy).

**Revendications** (pour les Etats contractants : BE, CH, LI, DE, FR, GB, IT, LU, NL, SE)

1. Phénylsulfonyloxy-benzimidazolyl-carbamates d'alkyles de formule I ci-dessous :

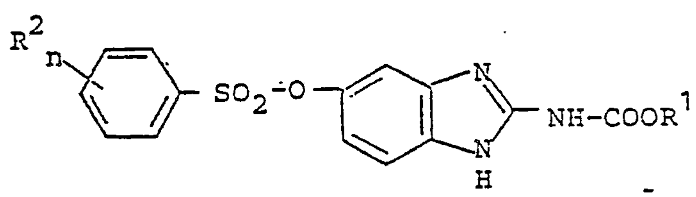

(I)

dans laquelle $R^1$ représente un alkyle à chaîne linéaire ou ramifiée en $C_1$ à $C_4$, n est le nombre 1 ou 2 et le ou les substituants $R^2$ désignent, indépendamment l'un de l'autre, le fluor, le groupe 1,1,2,2-tétrafluoroéthoxy ou un cyclo-alkyle en $C_3$ à $C_8$.

2. Composés de formule I selon la revendication 1 dans lesquels $R^1$ est le groupe méthyle, n le nombre 1 ou 2 et $R^2$ le fluor, le groupe 1,1,2,2-tétrafluoroéthoxy ou un cycloalkyle ayant 5, 6 ou 7 atomes de carbone.

3. Composé de formule I selon la revendication 1 dans lequel $R^1$ est le groupe méthyle, et $R^2$ un atome de fluor à la position 4.

4. Composé de formule I selon la revendication 1 dans lequel $R^1$ est le groupe méthyle et $R^2$ le groupe 1,1,2,2-tétrafluoroéthoxy à la position 3.

5. Procédé de préparation des composés de formule I selon la revendication 1, procédé caractérisé en ce que :

a) on fait réagir avec cyclisation un dérivé de o-phénylène-diamine de formule II :

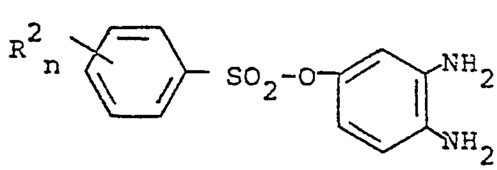

(II)

avec un dérivé d'un ester de l'acide carbamique de formule III :

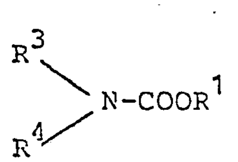

(III)

$R^1$ ayant la même signification que dans la formule I, $R^3$ désignant le groupe-CN et $R^4$ l'hydrogène, ou

bien $R^3$ et $R^4$ forment ensemble l'un des radicaux suivants, lié à l'atome d'azote par sa double liaison :

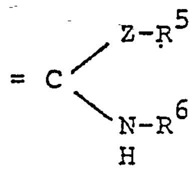 , 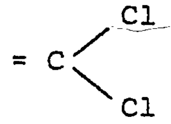 , 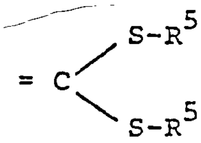

$R^5$ représentant un alkyle en $C_1$-$C_4$, $R^6$ l'hydrogène ou un groupe —$COOR^1$ et Z l'oxygène ou le soufre, ou bien

b) on fait réagir un benzimidazolyl-carbamate de formule IV :

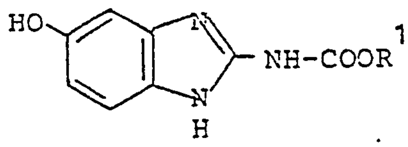

(IV)

avec un halogénure de benzène-sulfonyle substitué de formule V :

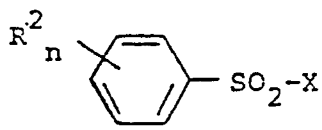

(V)

X désignant le fluor, le chlore, le brome ou l'iode, ou encore

c) on fait réagir un 2-aminobenzimidazole de formule VI :

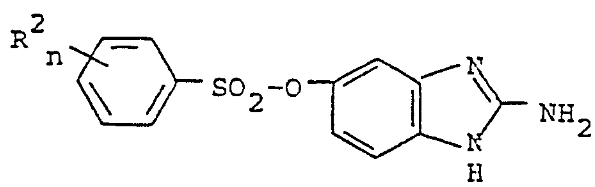

(VI)

avec un ester de l'acide carbonique de formule VII :

$$Y—COOR^1 \qquad (VII)$$

Y désignant un groupe —$OR^1$ ou un atome de chlore.

6. Agents de lutte contre des helminthiases caractérisés en ce qu'ils contiennent un phénylsulfonyloxybenzimidazolyl-carbamate de formule I selon la revendication 1.

**Revendications** (pour l'Etat contractant AT)

1. Procédé de préparation des phénylsulfonyloxy-benzimidazolyl-carbamates d'alkyles substitués de formule I ci-dessous :

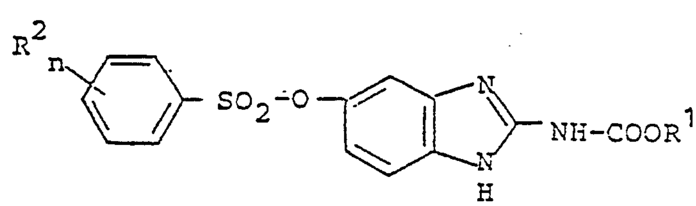

(I)

(dans laquelle $R^1$ représente un alkyle à chaîne linéaire ou ramifiée en $C_1$ à $C_4$, n est le nombre 1 ou 2 et le ou les substituants $R^2$ désignent, indépendamment l'un de l'autre, le fluor, le groupe 1,1,2,2-tétrafluoroéthoxy ou un cyclo-alkyle en $C_3$ à $C_8$), procédé caractérisé en ce que :

a) on fait réagir avec cyclisation un dérivé de o-phénylènediamine de formule II :

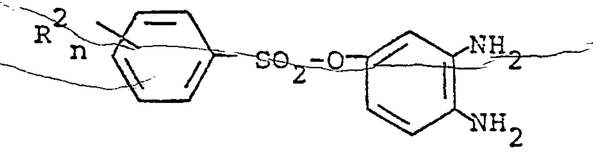

(II)

avec un dérivé d'un ester de l'acide carbamique de formule III :

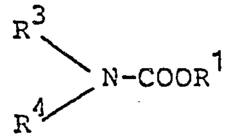

(III)

$R^1$ ayant la même signification que dans la formule I, $R^3$ désignant le groupe-CN et $R^4$ l'hydrogène, ou bien $R^3$ et $R^4$ forment ensemble l'un des radicaux suivants, lié à l'atome d'azote par sa double liaison :

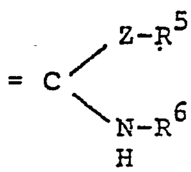 , 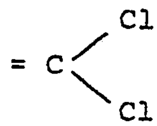 , 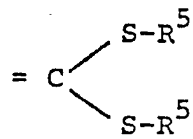

$R^5$ représentant un alkyle en $C_1$-$C_4$, $R^6$ l'hydrogène ou un groupe —$COOR^1$ et Z l'oxygène ou le soufre, ou bien

b) on fait réagir un benzimidazolyl-carbamate de formule IV :

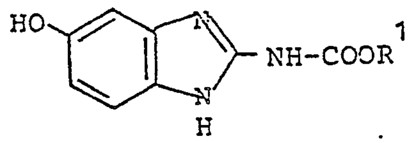

(IV)

avec un halogénure de benzène-sulfonyle substitué de formule V :

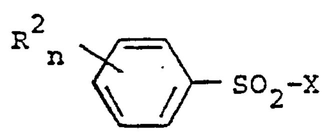

(V)

X désignant le fluor, le chlore, le brome ou l'iode, ou encore

c) on fait réagir un 2-aminobenzimidazole de formule VI :

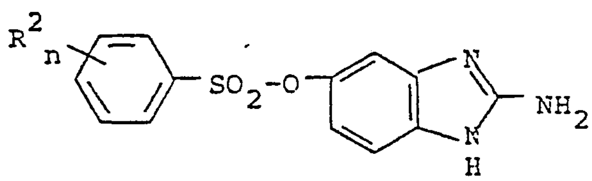

(VI)

avec un ester de l'acide carbonique de formule VII :

$$Y—COOR^1 \quad (VII)$$

Y désignant un groupe —$OR^1$ ou un atome de chlore.

2. Procédé selon la revendication 1 caractérisé en ce que dans les composés de formules I à III, $R^1$ est le groupe méthyle et $R^2$ le fluor, le groupe 1,1,2,2-tétrafluoroéthoxy ou un cycloalkyle ayant 5, 6 ou 7 atomes de carbone.

3. Procédé selon la revendication 1 ou 2 caractérisé en ce que dans les formules I à III, $R^1$ est le groupe méthyle et $R^2$ un atome de fluor à la position 4.

4. Procédé selon la revendication 1 ou 2 caractérisé en ce que dans les formules I à III, $R^1$ est le groupe méthyle et $R^2$ le groupe 1,1,2,2-tétrafluoroéthoxy à la position 3.